# EUROPEAN PATENT APPLICATION

(11) **EP 1 285 680 A1**
(43) Date of publication of application: **26.02.2003**
(21) Application number: 00929808.4
(22) Date of filing: 19.05.2000
(51) Int. Cl.: A61N 5/067

(54) **LASER BEAM APPLYING PROBE**

(71) Applicant: Yaman Ltd., Tokyo 135-0045 (JP)
(72) Inventor: Yamazaki, Iwao, Yaman Ltd., Tokyo 135-0045 (JP); Izawa, Yoshihiro, Yaman Ltd., Tokyo 135-0045 (JP)
(74) Representative: Newstead, Michael John
(86) International application number: PCT/JP00/03224
(87) International publication number: WO 01/087417

(57) **Abstract**

What is aimed at is to reduce the size of a laser beam projector to be small enough to be carried, and held and operated in single hand.

The hand-held casing 11 contains a circuit board 13 bearing a drive circuit and a control circuit for controlling intermittent radiation of the laser beam with the aid of an associated timer . The power supply is responsive to every and each depression of the push switch 16 on the front of the casing 11 for turning "on", changing the stretch of the "on" time step by step (1 to 6 seconds) and turning "off". The LED lamp 15 is responsive to the changing of the stretch of "on" time from 1 to 6 seconds for changing the color of the light from the LED lamp in the order that the light is green continuously; green intermittently; amber continuously; amber intermittently; red continuously and red intermittently. Final elongated (1.5 second-long) depression of the push switch 16 makes the power supply turn off, thereby stopping the raiation of the laser beam.

The single LED lamp permits identification of all stretches of "on" time, and the turning "on" and "off" of the power supply. This attributes elimination of the table-top casing, thus making the laser beam projector small and light enough to carry with ease.

## Description

### Technical Field:

The present invention relates to a laser beam projector for projecting a laser beam to a selected area on the skin for cosmetic treatment.

### Background Art:

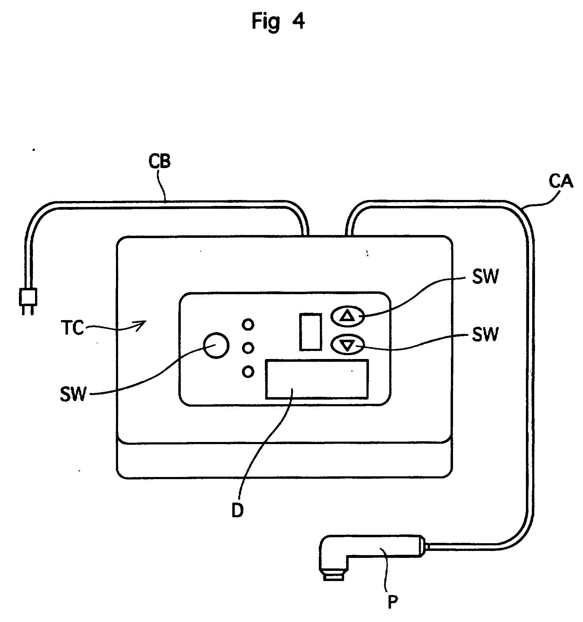

Fig.4 shows a conventional laser beam projector, which has its control section for controlling the intensity of the laser beam, the exposure period and other variable factors. The control section is provided as a separate unit. Specifically, the control section is contained in a table-top casing, and is connected to the laser beam projector via an associated cable CA. The table-top console TC is equipped with a liquid crystal display D and switches SW, thereby permitting the intensity of the laser beam, the exposure period and other variables to be controlled. Another length of cable CB is used in connecting the console TC to a wall outlet.

As may be understood, the console TC is a separate and relatively voluminous device, and the laser beam projector is relatively small, looking like an accessory part. The console-and-laser beam projector is large and heavy as a whole, and is inconvenient for carrying.

Installation of the console in the laser beam projector will attain the drastic down-sizing of the whole system, making it easy to carry to any desired place where a required cosmetic treatment can be effected at any time.

Also advantageously a person can handle the whole device in single hand to control all operations as required.

For the convenience of operation all switches are so placed that access by thumb may be permitted, and preferably push switches rather than rotary or snap ones are used; different modes of operation may be selected in respect of how many times one and same push switch is depressed. The liquid crystal display if attached to the front of the laser beam projector must be small enough not to occupy the substantial area. The permissible reduction of such display is limited to the extent that numeric figures and letters appearing in the display are easily noticeable to the eye. Required pieces of information are given in respect of different colors and frequency of the flickering of LED lamps.

In view of the above the object of the present invention is to provide a laser beam projector having all variables-setting, controlling and displaying functions installed in its body, and capable of presenting required pieces of information easily noticeable to the eye and of permitting all required operations to be effected by thumb in a single hand in which the laser beam projector is held.

### Disclosure of the Invention:

To attain the object of the present invention a laser beam projector is constructed according to claim 1 as follows:
it includes, in its body:
   a semiconductor laser diode for producing a laser beam;
   a drive circuit for driving the semiconductor laser;
   a heat sink for radiating the heat of the semiconductor laser and cooling the same;
   a CPU for controlling the on-time of the semiconductor laser;
   a condenser lens for collecting the laser beam from the semiconductor laser;
   a distance adjuster for adjusting the distance between a selected area on the skin and the focus point of the condenser lens; and
   an on-and-off power switch.

A laser beam projector of claim 1 is constructed according to claim 2 as follows:
the CPU is adapted to permit the intermittent throwing of the laser beam for a fixed recurrent on-period.

A laser beam projector of claim 2 is constructed according to claim 3 as follows:
the fixed recurrent on-period is determined step by step in respect of how many times a selected push switch is depressed.

A laser beam projector of claim 3 is constructed according to claim 4 as follows:
the selected push switch is the on-and-off power switch.

A laser beam projector of claim 4 is constructed according to claim 5 as follows:
the first depression of the selected push switch causes the power supply to turn on; subsequent depressions change the stretch of on-time step by step; and the final depression causes the power supply to turn off.

A laser beam projector of claim 5 is constructed according to claim 6 as follows:
different stretches of on-time are given in the form of three or more stepwise changes, stretches of on-time being sequentially elongated every time the push switch is depressed.

A laser beam projector of claim 6 is constructed according to claim 7 as follows:
it further includes an LED lamp to distinguish three or more different modes of operation.

A laser beam projector of claim 7 is constructed according to claim 8 as follows:
three or more different modes of operation are distinguished in respect of different colors of the LED lamp.

A laser beam projector of claim 7 is constructed according to claim 9 as follows:
three or more different modes of operation are distinguished in respect of whether the LED turns on or flickers.

A laser beam projector of claim 1 is constructed according to claim 10 as follows:
the on-and-off power switch is a switch accessible by the thumb in holding the laser beam projector in single hand.

A laser beam projector of claim 1 is constructed according to claim 11 as follows:
the on-and-off power switch is a power switch attached to the distance adjuster, which power switch is responsive to the touching of the distance adjuster on the selected area of the skin for turning on, thereby detecting the touching of the distance adjuster on the skin.

### Brief Description of Drawings:

Fig.1 is a front view of a laser beam projector according to the present invention;
Fig.2 is a longitudinal section of the laser beam projector of Fig.1;
Fig.3 illustrates how the LED display changes with the stretches of on-period; and
Fig.4 shows a conventional laser beam projector.

### Best Mode for Carrying Out the Invention:

Referring to the drawings, a laser beam projector according to one embodiment of the present invention is described below.

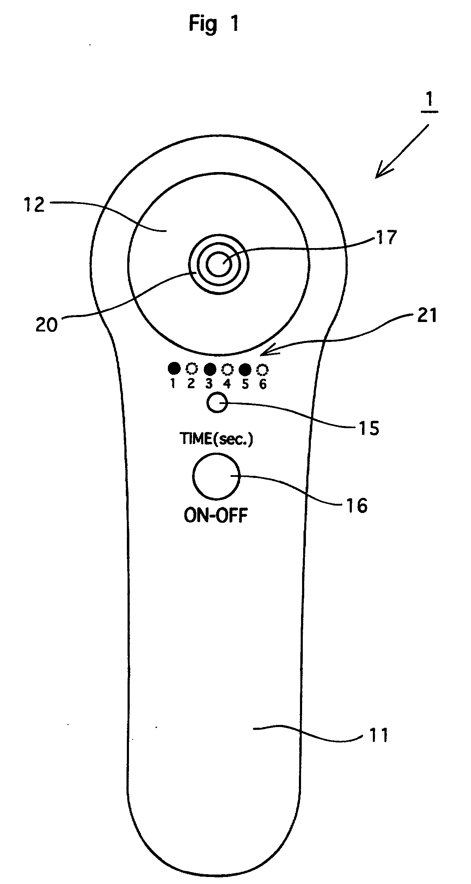

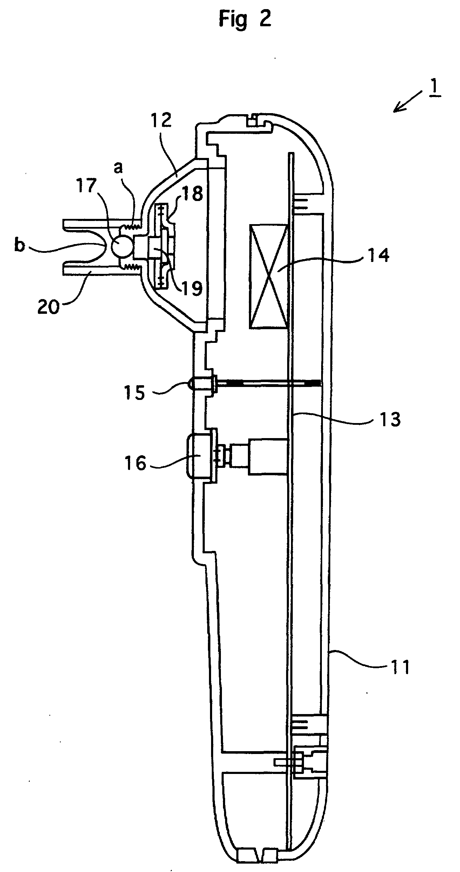

Figs.1 and 2 are a front view and longitudinal section of the laser beam projector.

The laser beam projector 1 has a head 12 projecting from its casing 11, and the casing 11 has a circuit bard 13 installed therein, which circuit board 13 bears a control circuit for controlling the stretch of on-period in intermittent radiation mode and a laser drive circuit (not shown). A cooling fan 14 is contained in the casing 11, and an LED lamp 15 and a push switch 16 are fixed to the front of the casing 11.

The LED lamp 15 is composed of red and green LED chips. When one or all LED chips turn simultaneously, the beam of red, green or yellow (or amber) light can be produced.

The cooling fan 14 is placed behind the head 12 to cool the same.

The LED lamp 15 and the push switch 16 are provided on the front side of the casing 11, as described earlier. Indication elements 21 describe what pieces of information are given by the LED lamp 15.

The head 12 has a spherical lens 17 fitted in its center hole, and a heat sink 18 is placed behind the spherical lens 17. The heat sink 18 has a semiconductor laser diode 19 inserted in its center hole.

The heat sink 18 deprives the semiconductor laser diode 19 of its heat by thermal conduction, thereby preventing the lowering of the performance of the semiconductor laser diode 19. The heat sink 18 is made of aluminum or an aluminum alloy, and it has some ducts made therein for improving the efficiency of thermal radiation.

The laser diode 19 may be a P-N junction diode of a chemical semiconductor such as GaAs (gallium arsenide), and it is driven by making an electric current flow directly in the P-N junction to produce a laser beam.

The laser diode 19 is small and light. It has a good stimulation efficiency, and is capable of working at a low voltage. The endurance life of the device is long, and it can be mass-produced, and accordingly it is less expensive. Also advantageously, it can be driven by using a battery. All of these make the laser diode most appropriate for use as a light source in a light, small, portable laser beam projector.

The head 12 has threads "a" formed on its front annular projection surrounding the spherical lens 17, and a circular adjuster 20 is screwed on the front annular projection.

The adjuster 20 is made of a transparent acrylic resin, thereby permitting the user to see from outside where the laser beam is projected on the skin.

The adjuster 20 has a notch "b" made on its circumference for ventilation.

The adjuster 20 functions as a spacer, taking a role of keeping the semiconductor laser diode 19 apart a given distance from the skin, the distance given by rotating the adjuster 20 around the threaded ring of the head 12.

The push switch 16 can be used in common for making the power supply turn on and for changing the stretches of intermittent radiation time. The electric power may be obtained from the wall outlet or from an installed battery.

A touch-responsive detection switch may be attached to the adjuster 20, thereby permitting the power supply to turn on in response to the touching of the adjuster 20 onto the skin, and turn off in response to departure of the adjuster 20 from the skin.

With this arrangement the laser beam cannot be produced unless the adjuster 20 is put on the skin, so that the laser beam projector may be guaranteed to be free of the danger for throwing a very hot narrow beam of light to the eye even if a person should inadvertently look into the adjuster opening. Thus, the safety is assured.

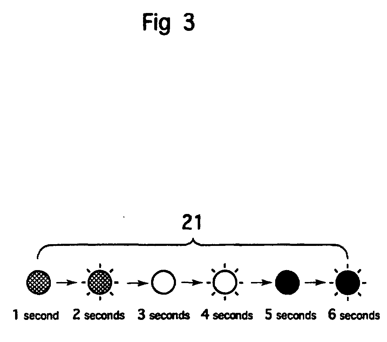

Referring to Fig.3, each and every push of the push switch 16 provides a series of different operations one after another, beginning with the turning-on of the power supply, followed by the resetting of the stretch of "on"-time (one to six second long stretch) and ending with the turning-off of the power supply.

The color of the light from the LED lamp 15 changes in the order of continuous green (1 second), intermittent green (2 seconds), continuous amber (3 seconds), intermittent amber (4 seconds), continuous red (5 seconds) and intermittent red (6 seconds). What the LED lamp 15 describes in terms of its color and lighting condition (continuous or intermittent lighting) can be easily understood by referring to which indication element 21 works. For examples, the LED lamp 15 produces a continuous red light, and then, the fifth indication element 21 informs that the stretch of "on"-time is 5 seconds long. The stretch of dormant time remains constant between those of "on" time (for example, 1 second).

Finally, the push switch 16 is depressed a relatively long time (1.5 or longer seconds) to make the power supply turn off, stopping radiation of the laser beam.

The stretch of "on"-time is limited by setting an associated timer for a relatively short length of time for example, 1 to 6 second-long period lest the skin should be injured by a single shot.

In use, first, the push switch 16 is depressed to turn the power supply on. Then, the semiconductor laser diode 19 turns on one second, and it turns off one second. This is repeated to produce an intermittent laser beam, making its radiation and dormancy appear alternately.

Repetitive depressions of the push switch 16 changes the stretch of "on" time one after another. When the desired "on" time is reached, depression of the push switch 16 stops.

Next, while radiating the laser beam, the tip end of the adjuster 20 is applied to a selected spot on the skin for treatment, effecting the required treatment while displacing the laser beam projector from place to place.

The focussing of the laser beam can be attained by rotating the adjuster 20 around the threaded ring "a".

### Industrial Adaptability:

As may be understood from the above, a laser beam projector according to the present invention contains all parts required for producing a laser beam in its body, and its console and display are provided on the front of the body.

Thus, the laser beam projector is portable, and therefore, it can be carried to a desired place where it can be used for treatment at any time.

A laser beam can be thrown intermittently for a fixed stretch of time, which can be changed step by step by depressing the power push switch repeatedly.

Accordingly all operations required for radiation of a laser beam can be performed simply by depressing the multi-function switch while holding the laser beam projector in one hand, thus eliminating the necessity of using a table-top casing.

Conveniently repetitive depression of the single push switch permits all required operations to be selectively performed, and accordingly the number of the parts and hence, the manufacturing cost is reduced.

Repetitive depression of the single push switch extends the stretch of "on"-time step by step.

Thus, the operation modes can be selected one after another by depressing the push switch repetitively while feeling what strong effect the laser beam is causing on the skin. This facilitates the controlling of treatment.

Three or more modes of operation can be identified in terms of whether the LED lamp turns on continuously or intermittently, and of what color is the beam of light from the LED lamp.

Thus, the single LED lamp informs the user of which mode of operation the laser beam projector is working. Advantageously, this arrangement requires an occupation space much smaller than an LC panel used as a display, and is simple and less expensive.

## Claims

1. A laser beam projector comprising: in its body,
a semiconductor laser diode for producing a laser beam;
a drive circuit for driving the semiconductor laser;
a heat sink for radiating the heat of the semiconductor laser and cooling the same;
a CPU for controlling the on-time of the semiconductor laser;
a condenser lens for collecting the laser beam from the semiconductor laser;
a distance adjuster for adjusting the distance between a selected area on the skin and the focus point of the condenser lens; and
an on-and-off power switch.

2. A laser beam projector according to claim 1 wherein the CPU is adapted to permit the intermittent throwing of the laser beam for a fixed recurrent on-period.

3. A laser beam projector according to claim 2 wherein the fixed recurrent on-period is determined step by step in respect of how many times a selected push switch is depressed.

4. A laser beam projector according to claim 3 wherein the selected push switch is the on-and-off power switch.

5. A laser beam projector according to claim 4 wherein the first depression of the selected push switch causes the power supply to turn on; subsequent depressions change the stretch of on-time step by step; and the final depression causes the power supply to turn off.

6. A laser beam projector according to claim 5 wherein different stretches of on-time are given in the form of three or more stepwise changes, stretches of on-time being sequentially elongated every time the push switch is depressed.

7. A laser beam projector according to claim 6 wherein it further includes an LED lamp to distinguish three or more different modes of operation.

8. A laser beam projector according to claim 7 wherein three or more different modes of operation are distinguished in respect of different colors of the LED lamp.

9. A laser beam projector according to claim 7 wherein three or more different modes of operation are distinguished in respect of whether the LED LAMP turns on or flickers.

10. A laser beam projector according to claim 1 wherein the on-and-off power switch is a switch accessible by the thumb in holding the laser beam projector in single hand.

11. A laser beam projector according to claim 1 wherein the on-and-off power switch is a power switch attached to the distance adjuster, which power switch is responsive to the touching of the distance adjuster on the selected area of the skin for turning on, thereby detecting the touching of the distance adjuster on the skin.
